# EUROPEAN PATENT APPLICATION

(11) **EP 4 446 418 A1**
(43) Date of publication of application: **16.10.2024**
(21) Application number: 22904185.0
(22) Date of filing: 05.12.2022
(51) Int. Cl.: C12N 15/864, A61K 35/76, A61K 48/00, A61P 1/00, A61P 13/12, A61P 17/00, A61P 25/00, A61P 27/02, A61P 27/16, A61P 35/00, C07K 14/015, C12N 15/09, C12N 15/12, C12Q 1/6897, C12Q 1/70

(54) **MODIFIED ADENO-ASSOCIATED VIRUS VECTOR**

(30) Priority: 06.12.2021 JP 2021198101
(71) Applicant: Juntendo Educational Foundation, Tokyo 113-8421 (JP)
(72) Inventor: KAMIYA Kazusaku, Tokyo 113-8421 (JP)
(74) Representative: Schwarz & Partner Patentanwälte GmbH
(86) International application number: PCT/JP2022/044701
(87) International publication number: WO 2023/106256

(57) **Abstract**

As a modified AAV vector having excellent infection directivity into epithelial cells represented by inner-ear epithelial cells, provided is a modified adeno-associated virus vector in which some amino acid residues in the capsid sequence of a wild-type adeno-associated virus are substituted with other amino acid residues, in which one or more amino acid residues selected from (1) position 129 (Leu), (2) position 268 (Ser), (3) position 447 (Asn), (4) position 470 (Gly), (5) position 472 (Ser), (6) position 473 (Thr), (7) position 502 (Thr), and (8) position 531 (Glu) of the capsid sequence of AAV1 of SEQ ID NO: 1 or an amino acid residue at a position corresponding thereto is substituted with other amino acid residues.

## Description

### Technical Field

The present invention relates to modified adeno-associated virus vectors.

### Background Art

There are various serotypes of adeno-associated virus (AAV) used as a gene therapy vector (such as AAV1-10). The infection directivity of AAV greatly varies depending on the type of serotype, and it is necessary to select an optimal vector depending on the target cell of gene therapy or genome editing therapy to be targeted. However, the existing AAV vectors do not necessarily show optimal infection directivity to target cells. Since the infection directivity of AAV varies depending on the amino acid sequence of the capsid region, it is possible to develop a vector having optimum infection directivity by modifying the same sequence. From such a viewpoint, modified adeno-associated virus vectors for gene therapy and genome editing therapy for various diseases have been studied.

Wild serotypes such as AAV1 and AAV2 have been used as candidates for gene therapy to the inner ear, but capsid-modified vectors such as AAV-ANC80 L65 (Patent Literature 1) have recently been developed. These can be transfected into the inner ear, but have different infection directivity. For example, AAV1 is capable of infecting cochlear ectodermal cells (outer spiral groove cells), but is less capable of infecting other epithelial cells. AAV2 has high infectivity to intracochlear hair cells, but has weak infectivity to other epithelial cells. The AAV-ANC80 L65 vector having an increased ability to infect the inner ear of AAV2 has an ability to infect a part of the outer hair cells in addition to the ability to infect the inner hair cells possessed by AAV2, but has a low ability to infect cochlear external cells and cochlear internal cells (inner spiral groove cells) having a gap junction network, and thus, it is difficult to apply the AAV-ANC80 L65 vector to gene therapy for GJB2 mutant deafness (characterized by gap junction aplasia), which is the most prevalent in genetic deafness.

### Citation List

### Patent Literature

Patent Literature 1: WO 2007/019994 A

### Non Patent Literature

Non Patent Literature 1: Journal of Virology,2016,90(11):5219-5230

### Summary of Invention

### Technical Problem

As described above, a modified AAV vector having excellent infection directivity to epithelial cells represented by inner-ear cells, stromal cells, neuronal cells, retinal cells, lens cells, cancer cells, skin cells, and cells expressing gap junctions and connexin molecules of components has not yet been developed.

Therefore, an object of the present invention is to provide a modified AAV vector excellent in infection directivity to epithelial cells typified by inner-ear cells, stromal cells, neuronal cells, retinal cells, lens cells, cancer cells, skin cells, and cells expressing gap junctions and connexin molecules.

### Solution to Problem

Therefore, the present inventor first examined the infection directivity of the serotype of AAV to the inner-ear epithelial cells, and found that AAV1, AAV6, AAV7, and AAV8 have high infection directivity to the inner-ear epithelial cells, particularly to the supporting cells forming the inner-ear gap junction. Therefore, as a result of partially modifying the amino acid sequence of these wild-type AAV1, AAV6, AAV7, AAV8, and the like, it has been found that when an amino acid residue at a specific position is substituted with another amino acid residue, a vector having a different infection directivity from the AAV-ANC80 L65 vector described in Patent Literature 1 and other wild-type AAV, and having a high infection directivity to epithelial cells represented by inner-ear epithelial cells has been discovered, and the present invention has been completed.

That is, the present invention provides the following inventions [1] to [17].
[1] A modified adeno-associated virus vector in which some amino acid residues in the capsid sequence of adeno-associated virus are substituted with other amino acid residues, wherein one or more amino acid residues selected from (1) position 129 (Leu), (2) position 268 (Ser), (3) position 447 (Asn), (4) position 470 (Gly), (5) position 472 (Ser), (6) position 473 (Val), (7) position 502 (Thr), and (8) position 531 (Glu) or an amino acid residue at a position corresponding thereto in alignment with the amino acid sequence of the capsid sequence of AAV1 of SEQ ID NO: 1 are substituted with other amino acid residues.
[2] The modified adeno-associated virus vector according to [1], in which the substitution of the amino acid residue is one or more selected from Leu129Phe, Ser268Thr, Asn447Ser, Gly470Thr, Ser472Ala, Val473Asn, Thr502Ala, and Glu531Lys.
[3] The modified adeno-associated virus vector according to [1] or [2], which is a vector having improved infection directivity of epithelial cells to target cells.
[4] The modified adeno-associated virus vector according to [3], in which the epithelial cells are epithelial cells selected from inner-ear epithelial cells, intestinal epithelial cells, retinal cells, skin cells, renal cells, airway epithelial cells, neuronal cells, cardiomyocytes, and cancer cells in which Connexin expression is involved.
[5] The modified adeno-associated virus vector according to [3] or [4], in which the epithelial cell is an inner-ear epithelial cell.
[6] The modified adeno-associated virus vector according to any one of [1] to [5], further incorporating a gene that can be applied to gene therapy or genome editing therapy for genetic diseases, tissue damage, and cancers selected from GJB2 gene, SLC26A4 gene, TMC1 gene, TMC2 gene, and Atoh1 gene.
[7] A gene therapeutic agent or a genome editing therapeutic agent for treating a genetic disease, tissue damage, or cancer in a target cell, containing the modified adeno-associated virus vector according to [6].
[8] Use of the modified adenovirus-associated vector according to [6] for producing a gene therapeutic agent or a genome editing therapeutic agent for treating a genetic disease, tissue damage, or cancer in a target cell.
[9] The modified adenovirus-associated vector according to [6], for use in the treatment of a genetic disease, tissue damage, or cancer in a target cell.
[10] A treatment method of a genetic disease, tissue damage or cancer in a target cell, the treatment method including administering the modified adeno-associated virus vector according to [6].
[11] A producing method of a modified adeno-associated virus vector having improved infection directivity to a target cell, the producing method including a process of substituting any one or more amino acid residues selected from the following (a) with other amino acid residues in the amino acid sequence of the capsid sequence:
   (a) in alignment with the amino acid sequence of the capsid sequence of AAV1 of SEQ ID NO: 1, an amino acid residue at a position corresponding to
      (1) position 129 (Leu),
      (2) position 268 (Ser),
      (3) position 447 (Asn),
      (4) position 470 (Gly),
      (5) position 472 (Ser),
      (6) position 473 (Val),
      (7) position 502 (Thr), and
      (8) position 531 (Glu).
[12] The producing method according to [11], further including
   a process of substituting any one or more amino acid residues selected from the following (a) with other amino acid residues in the amino acid sequence of the capsid sequence of the reference adeno-associated virus vector to prepare a candidate modified adeno-associated virus vector,
      (a) in alignment with the amino acid sequence of the capsid sequence of AAV1 of SEQ ID NO: 1, an amino acid residue at a position corresponding to
         (1) position 129 (Leu),
         (2) position 268 (Ser),
         (3) position 447 (Asn),
         (4) position 470 (Gly),
         (5) 472 (Ser),
         (6) 473 (Val),
         (7) 502 (Thr), and
         (8) 531 (Glu);
   a process of measuring and evaluating the infectious dose of the candidate-modified adeno-associated virus vector with the target cell by the number of positive cells for the reporter gene or the number of positive cells for the expressed protein; and
   a process of selecting a candidate modified adeno-associated virus vector in which the infectious dose with the target cell is increased as compared to the reference adeno-associated virus vector before introducing the amino acid substitution.
[13] The producing method according to [12], in which the reference adeno-associated virus vector is AAV1, AAV6, AAV7, or AAV8.
[14] The producing method according to [12], in which the amino acid substitution is one or more selected from the group consisting of
   Leu129Phe,
   Ser268Thr,
   Asn447Ser,
   Gly470Thr,
   Ser472Ala,
   Val473Asn,
   Thr502Ala, and
   Glu531Lys.
[15] The producing method according to [12], in which the target cell is a cell of the inner ear, skin, heart, eye, brain, upper gastrointestinal tract, lower gastrointestinal tract, or kidney.
[16] The producing method according to [12], in which the target cell is a cell selected from inner-ear epithelial cells, intestinal epithelial cells, airway epithelial cells, retinal cells, lens cells, skin cells, renal cells, airway epithelial cells, neuronal cells, cardiomyocytes, stromal cells, and cancer cells in which Connexin expression is involved.
[17] The producing method according to [12], in which the target cell is an inner-ear epithelial cell.

In addition, as another aspect, the present invention also provides the following inventions [1] to [17].
[1] A modified adeno-associated virus vector exhibiting improved infectivity tropism for a target cell, in which a substitution of one or two or more amino acid residues selected from the following (1) to (8) is introduced into the amino acid sequence of the capsid sequence.
   (1) A substitution of leucine present at a position corresponding to position 129 of the amino acid sequence of SEQ ID NO: 1 with another amino acid residue in alignment with the amino acid sequence of SEQ ID NO: 1,
   (2) A substitution of serine present at a position corresponding to position 268 of the amino acid sequence of SEQ ID NO: 1 with another amino acid residue in alignment with the amino acid sequence of SEQ ID NO: 1,
   (3) A substitution of an asparagine present at a position corresponding to position 447 of the amino acid sequence of SEQ ID NO: 1 with another amino acid residue in alignment with the amino acid sequence of SEQ ID NO: 1,
   (4) A substitution of glycine present at a position corresponding to position 470 of the amino acid sequence of SEQ ID NO: 1 with another amino acid residue in alignment with the amino acid sequence of SEQ ID NO: 1,
   (5) A substitution of serine present at the position corresponding to position 472 of the amino acid sequence of SEQ ID NO: 1 with another amino acid residue in alignment with the amino acid sequence of SEQ ID NO: 1,
   (6) A substitution of valine present at the position corresponding to position 473 of the amino acid sequence of SEQ ID NO: 1 with another amino acid residue in alignment with the amino acid sequence of SEQ ID NO: 1,
   (7) A substitution of a threonine present at the position corresponding to position 502 of the amino acid sequence of SEQ ID NO: 1 with another amino acid residue in alignment with the amino acid sequence of SEQ ID NO: 1, and
   (8) A substitution of a glutamic acid present at the position corresponding to position 531 of the amino acid sequence of SEQ ID NO: 1 with another amino acid residue in alignment with the amino acid sequence of SEQ ID NO: 1.
[2] The modified adeno-associated virus vector according to [1], in which the substitution of the amino acid residues is
   (1) a substitution of leucine present at a position corresponding to position 129 of the amino acid sequence of SEQ ID NO: 1 with phenylalanine in alignment with the amino acid sequence of SEQ ID NO: 1,
   (2) a substitution of serine present at a position corresponding to position 268 of the amino acid sequence of SEQ ID NO: 1 with threonine in alignment with the amino acid sequence of SEQ ID NO: 1,
   (3) a substitution of asparagine present at a position corresponding to position 447 of the amino acid sequence of SEQ ID NO: 1 with serine in alignment with the amino acid sequence of SEQ ID NO: 1,
   (4) a substitution of glycine present at a position corresponding to position 470 of the amino acid sequence of SEQ ID NO: 1 with threonine in alignment with the amino acid sequence of SEQ ID NO: 1,
   (5) a substitution of serine present at the position corresponding to position 472 of the amino acid sequence of SEQ ID NO: 1 with alanine in alignment with the amino acid sequence of SEQ ID NO: 1,
   (6) a substitution of valine present at the position corresponding to position 473 of the amino acid sequence of SEQ ID NO: 1 with asparagine in alignment with the amino acid sequence of SEQ ID NO: 1,
   (7) a substitution of threonine present at the position corresponding to position 502 of the amino acid sequence of SEQ ID NO: 1 with alanine in alignment with the amino acid sequence of SEQ ID NO: 1, or
   (8) a substitution of glutamic acid present at the position corresponding to position 531 of the amino acid sequence of SEQ ID NO: 1 with leucine in alignment with the amino acid sequence of SEQ ID NO: 1.
[3] The modified adeno-associated virus vector according to [1] or [2], having substitutions of the amino acid residues of (2) to (7) above in combination.
   [3-2] The modified adeno-associated virus vector according to [3], having a capsid sequence consisting of the amino acid sequence of SEQ ID NO: 14 or SEQ ID NO: 15.
[4] The modified adeno-associated virus vector according to [3], further having a substitution of the amino acid residue in (1) or (8) above.
   [4-2] The modified adeno-associated virus vector according to [4], having a capsid sequence consisting of the amino acid sequence of SEQ ID NO: 16 or SEQ ID NO: 17.
   [4-3] The modified adeno-associated virus vector according to [1] or [2], having substitutions of the amino acid residues of (3) to (7) above in combination.
   [4-4] The modified adeno-associated virus vector according to [4-3], having a capsid sequence consisting of the amino acid sequence of SEQ ID NO: 18.
[5] The modified adeno-associated virus vector according to any one of [1] to [4], in which the target cell is an epithelial cell, a stromal cell, a lens cell, or a cancer cell.
[6] The modified adeno-associated virus vector according to [5], in which the epithelial cell is an inner-ear epithelial cell, an intestinal epithelial cell, a retinal cell, a skin cell, a renal cell, an airway epithelial cell, a neuronal cell, or a uterine cell.
[7] The modified adeno-associated virus vector according to [5] or [6], in which the target cell has a gap junction or expresses a connexin molecule constituting a gap junction.
[8] The modified adeno-associated virus vector according to [7], in which genes that cause genetic diseases selected from connexin 26 (GJB2) gene, pendrin (SLC26A4) gene, TMC1 gene, TMC2 gene, voltage-dependent potassium channel subfamily Q member 4) (KCNQ4), cadherin 23 (CHD23) gene, protocadherin 15 (PCDH15) gene, and otoferlin (OTOF) gene, gene sequences for genome editing thereof, protein atonal homolog 1 (Atoh1) gene, cyclin dependent kinase inhibitor 1B (CDKN1B) gene, and basic helix-loop-helix type transcription factor (Hes1) gene are incorporated.
[9] The modified adeno-associated virus vector according to [8], in which a promoter containing any of the base sequences of SEQ ID NO: 11 to 13 is incorporated upstream of GJB2 gene so that GJB2 gene can be expressed.
   [9-2] The modified adeno-associated virus vector according to [9], in which an enhancer containing a base sequence of SEQ ID NO: 19 is incorporated upstream of GJB2 gene to activate the promoter.
[10] A medicament containing the modified adeno-associated virus vector according to any one of [1] to [9].
[11] The medicament according to [10], for use in a treatment of a genetic disease, tissue damage and/or cancer, or in genome editing.
[12] Use of the modified adeno-associated virus vector according to any one of [1] to [9] for a manufacture of a medicament for a genetic disease, tissue damage and/or cancer, or genome editing.
[13] A method for treating a genetic disease, tissue damage and/or cancer, the method including a step of administering the modified adeno-associated virus vector according to any one of [1] to [9] to a subject having a genetic disease, tissue damage and/or cancer in a therapeutically effective amount.
[14] A genome editing method including a step of administering the modified adeno-associated virus vector according to any one of [1] to [9] to a subject in an effective amount.
[15] A producing method of a modified adeno-associated virus vector exhibiting improved infectivity tropism to a target cell, the producing method including
   a process of introducing substitutions of one or two or more amino acid residues selected from the following (1) to (8) into the amino acid sequence of the capsid sequence.
   (1) A substitution of leucine present at a position corresponding to position 129 of the amino acid sequence of SEQ ID NO: 1 with another amino acid residue in alignment with the amino acid sequence of SEQ ID NO: 1,
   (2) A substitution of serine present at a position corresponding to position 268 of the amino acid sequence of SEQ ID NO: 1 with another amino acid residue in alignment with the amino acid sequence of SEQ ID NO: 1,
   (3) A substitution of an asparagine present at a position corresponding to position 447 of the amino acid sequence of SEQ ID NO: 1 with another amino acid residue in alignment with the amino acid sequence of SEQ ID NO: 1,
   (4) A substitution of glycine present at a position corresponding to position 470 of the amino acid sequence of SEQ ID NO: 1 with another amino acid residue in alignment with the amino acid sequence of SEQ ID NO: 1,
   (5) A substitution of serine present at the position corresponding to position 472 of the amino acid sequence of SEQ ID NO: 1 with another amino acid residue in alignment with the amino acid sequence of SEQ ID NO: 1,
   (6) A substitution of valine present at the position corresponding to position 473 of the amino acid sequence of SEQ ID NO: 1 with another amino acid residue in alignment with the amino acid sequence of SEQ ID NO: 1,
   (7) A substitution of a threonine present at the position corresponding to position 502 of the amino acid sequence of SEQ ID NO: 1 with another amino acid residue in alignment with the amino acid sequence of SEQ ID NO: 1, and
   (8) A substitution of a glutamic acid present at the position corresponding to position 531 of the amino acid sequence of SEQ ID NO: 1 with another amino acid residue in alignment with the amino acid sequence of SEQ ID NO: 1.
[16] The producing method according to [15], further including
   a process of introducing substitutions of one or two or more amino acid residues selected from the above (1) to (8) into the amino acid sequence of the capsid sequence of the reference adeno-associated virus vector to prepare a candidate adeno-associated virus vector,
   a process of measuring an infectious dose of the target cell of the candidate adeno-associated virus vector, and
   a process of selecting a candidate adeno-associated virus vector in which the infectious dose of the target cell is increased as compared to the reference adeno-associated virus vector.
[17] The producing method according to [15], wherein the reference adeno-associated virus vector is AAV1 to AAV10, preferably AAV1, AAV6, AAV7, or AAV8, more preferably AAV1 or AAV6, particularly preferably AAV1.

### Advantageous Effects of Invention

Since the modified AAV vector of the present invention has high infection directivity to epithelial cells represented by inner-ear epithelial cells, it is useful as a gene therapeutic agent, a genome editing therapeutic agent, or a vector for regenerative medicine for various diseases caused by abnormalities of these epithelial cells. In particular, the modified AAV vector of the present invention in which the GJB2 gene is incorporated is useful as a deafness gene therapeutic agent.

### Brief Description of Drawings

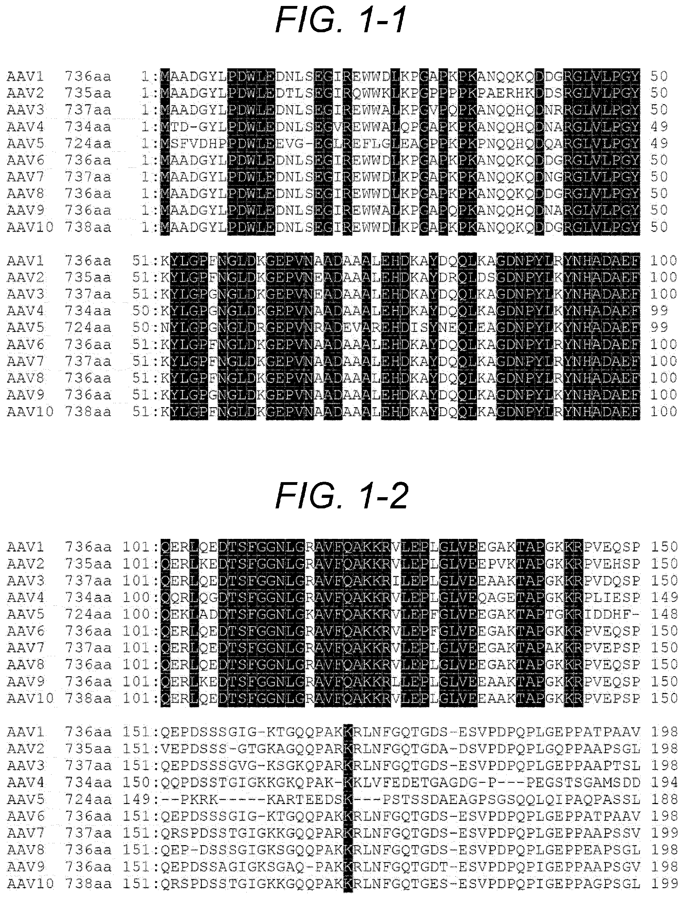

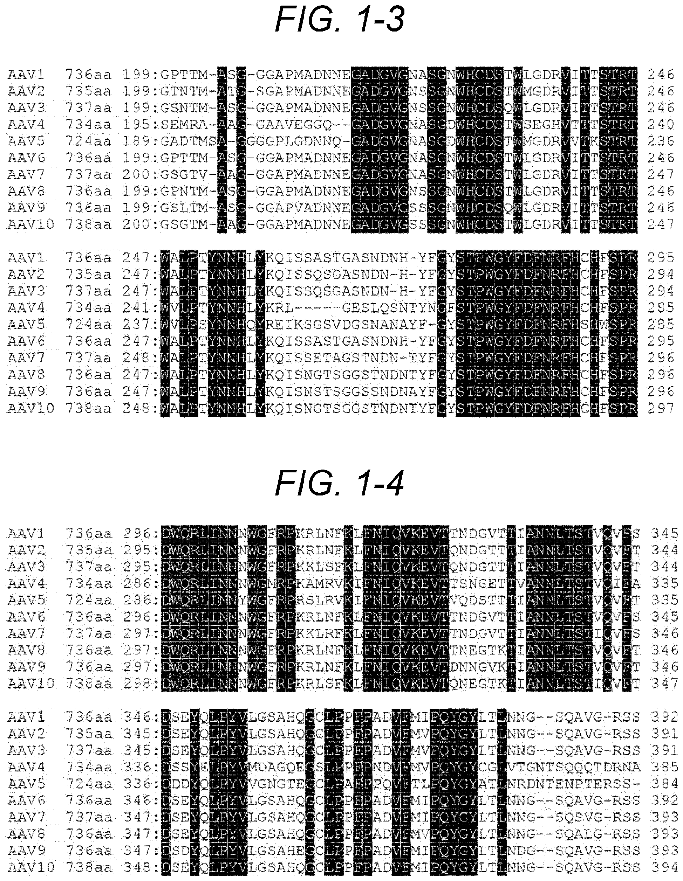

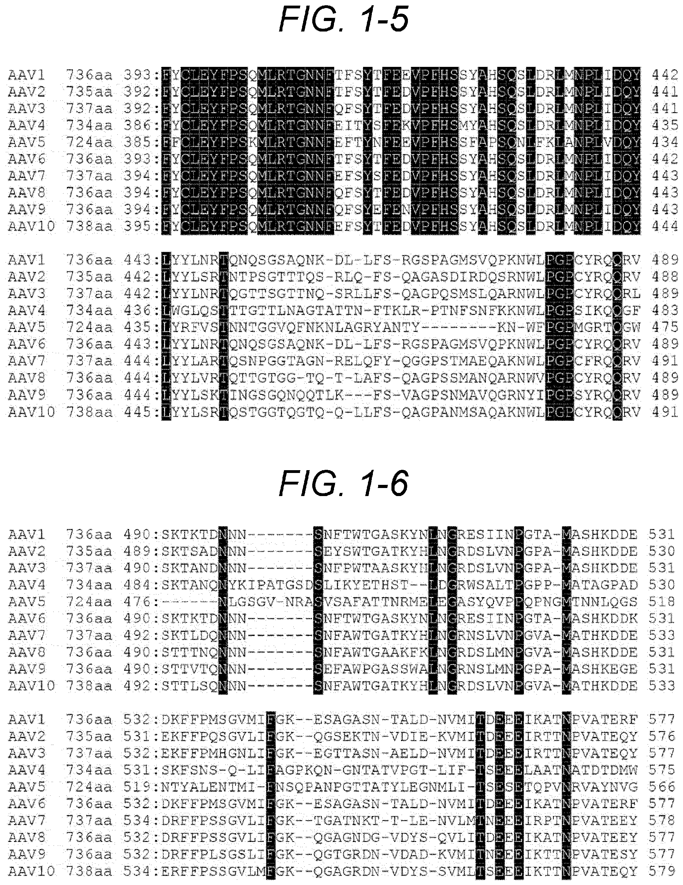

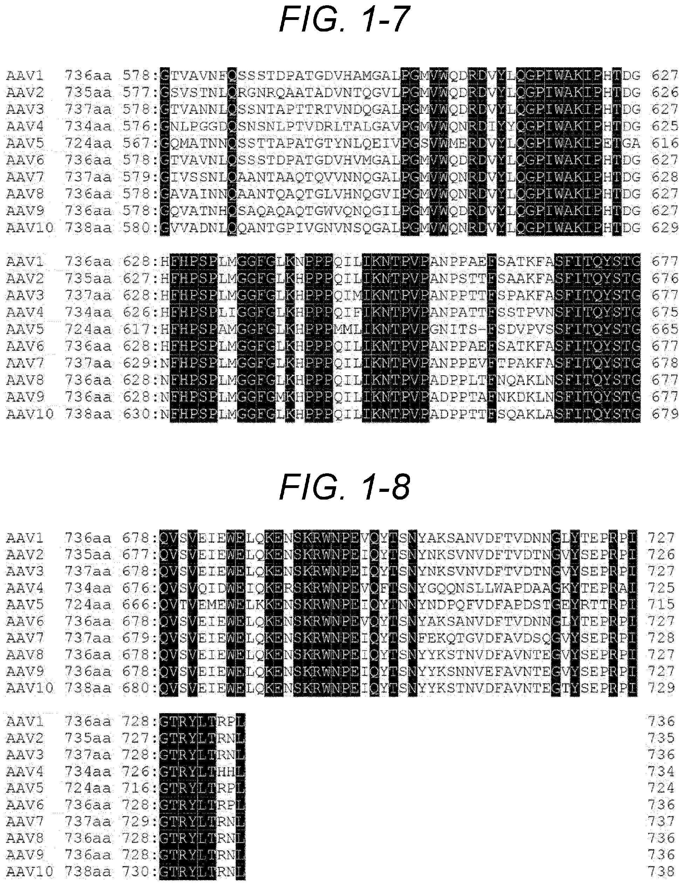

Fig. 1-1 is a diagram illustrating alignment of capsid sequences of AAV1-10.
Fig. 1-2 is a diagram illustrating alignment of the capsid sequences of AAV1-10.
Fig. 1-3 is a diagram illustrating alignment of the capsid sequences of AAV1-10.
Fig. 1-4 is a diagram illustrating alignment of the capsid sequences of AAV1-10.
Fig. 1-5 is a diagram illustrating alignment of the capsid sequences of AAV1-10.
Fig. 1-6 is a diagram illustrating alignment of the capsid sequences of AAV1-10.
Fig. 1-7 is a diagram illustrating alignment of the capsid sequences of AAV1-10.
Fig. 1-8 is a diagram illustrating alignment of the capsid sequences of AAV1-10.
Fig. 2 is a diagram illustrating infection directivity of AAV into inner-ear cells of various serotypes.
Fig. 3 is a diagram quantifying the infection directivity of AAV to inner-ear cells of various serotypes.
Fig. 4 illustrates a stained image of a tissue obtained by introducing a modified adeno-associated virus vector in which GJB2 is introduced by AAV-sia1.8 into the inner ear epithelium of a GJB2 deficient mouse. Red indicates connexin 26 protein by introduced GJB2 gene. Green indicates EGFP reporter.
Fig. 5 illustrates a stained image of a tissue obtained by introducing a modified adeno-associated virus vector in which GJB2 is introduced by AAV-sia6.8 into the inner ear epithelium of a GJB2 deficient mouse. Red indicates gap junction by introduced GJB2 gene, and green indicates reporter of EGFP. Portions with red lines between cells are gap junctions formed by gene therapy.
Fig. 6 illustrates stained images of cultured cochlear organs of GJB2 deficient mice infected with wild-type AAV6 carrying a GJB2 gene and an EGFP gene and AAV-sia6.8. Red indicates connexin 26 protein that is an expression product of GJB2 gene. Green indicates EGFP reporter.
Fig. 7 is a schematic view illustrating cells constituting a cochlear organ.
Fig. 8 illustrates EGFP-positive area ratios in a region of outer spiral groove cells (OSC) and a region containing outer hair cells and diterus cells (OHC+DC).
Fig. 9 illustrates the results of detecting the expression of connexin 26 (CX26) protein and connexin 30 (CX30) protein by immunostaining inner-ear epithelial cells derived from iPSCs of deafness patients infected with AAV-sia6.8 carrying a wild-type GJB2 gene.
Fig. 10 illustrates an expression intensity ratio of CX30 protein to CX26 protein.
Fig. 11 illustrates cultured cochlear organs of mice infected with AAV-sia6.8 carrying protein atonal homologue 1 (Atoh1) gene.
Fig. 12 illustrates threshold values of ABR of GJB2 deficient mice treated with AAV-sia6.8 carrying a wild-type GJB2 gene before surgery, 1 week after surgery, and 2 weeks after surgery.
Fig. 13 illustrates waveforms of ABR of GJB2 deficient mice treated with AAV-sia6.8 carrying a wild-type GJB2 gene before surgery, 1 week after surgery, and 2 weeks after surgery.
Fig. 14 illustrates stained images of cultured cochlear organs of GJB2 deficient mice into which promoter 2 has been introduced and which have been infected with AAV-sia6.8 carrying a GJB2 gene and an EGFP gene. Red indicates gap junction by introduced GJB2 gene, and green indicates reporter of EGFP.
Fig. 15 is a stained image of a cultured cochlear organ of a mouse infected with AAV-sia6.8 carrying EGFP gene and a Thr268Ser variant thereof.
Fig. 16 illustrates EGFP-positive area ratios in a region of outer spiral groove cells (OSC) and a region containing outer hair cells and diterus cells (OHC+DC).

### Description of Embodiments

Wild-type AAV includes AAV1 to AAV10 as serotypes.

Although not particularly limited, AAV1, AAV6, AAV7, and AAV8 are preferably used, AAV1, AAV6, or AAV8 is more preferably used, AAV1 or AAV6 is still more preferably used, and AAV1 is still more preferably used as the "reference AAV" into which an amino acid residue substitution is to be introduced.

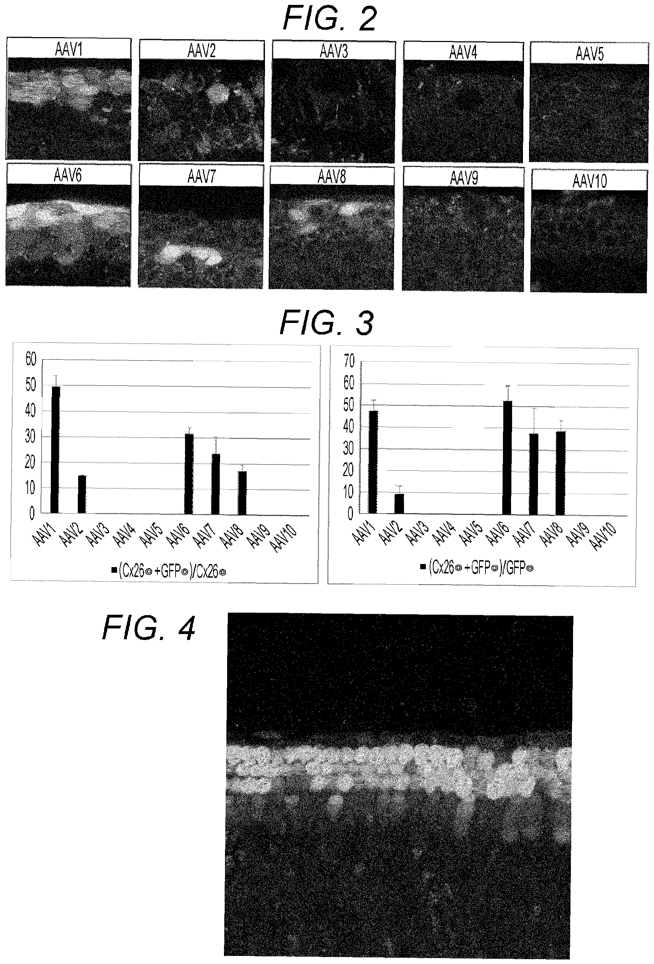

As illustrated in Figs. 2 and 3, by the test using various AAV vectors of the present inventors, it was found that AAV1, AAV6, AAV7, and AAV8 have a higher infection directivity to inner-ear cells, particularly to supporting cells forming gap junctions, than other serotypes. Therefore, it is preferable to use the reference AAV for modification of AAV1, AAV6, AAV7, and AAV8.

The modified AAV vector of the present invention is a modified adeno-associated virus vector in which some amino acid residues of the capsid sequence of the wild adeno-associated virus are substituted with other amino acid residues, and a modified AAV vector in which some amino acid residues of the capsid sequence of AAV1, AAV6, AAV7, or AAV8 are substituted with other amino acid residues is preferable. In addition, a modified AAV vector in which some amino acid residues of the capsid sequence of AAV1, AAV6, or AAV8 are substituted with other amino acid residues is more preferable, a modified AAV vector in which some amino acid residues of the capsid sequence of AAV1 or AAV6 are substituted with other amino acid residues is still more preferable, and a modified AAV vector in which some amino acid residues of the capsid sequence of AAV1 are substituted with other amino acid residues is still more preferable.

More specifically, in alignment with the amino acid sequence of the capsid sequence of AAV1 of SEQ ID NO: 1, a modified adeno-associated virus vector in which one or more amino acid residues selected from (1) position 129 (Leu), (2) position 268 (Ser), (3) position 447 (Asn), (4) position 470 (Gly), (5) position 472 (Ser), (6) position 473 (Thr), (7) position 502 (Thr), and (8) position 531 (Gln) or an amino acid residue at a position corresponding thereto are substituted with another amino acid residue is preferable.

It was revealed that the amino acid residues present at the positions defined in (1) to (8) above play an important role in determining the infectivity tropism of AAV to target cells. Therefore, by substituting the amino acid residues present at these positions with other amino acid residues, the infectivity tropism of AAV to the target cell can be changed, and it can be expected that a modified AAV exhibiting improved infectivity tropism to the target cell of interest can be obtained.

Therefore, the present invention provides a producing method of modified AAVs that exhibit improved infectivity tropism toward target cells, the producing method including
a process of introducing substitutions of one or two or more amino acid residues selected from the above (1) to (8) into the amino acid sequence of the capsid sequence of a reference AAV to prepare a candidate AAV (candidate AAV preparation process),
a process of measuring an infectious dose of the target cells of the candidate AAV (measuring process), and
a process of selecting a candidate AAV in which the infection amount of the target cell is increased as compared with the reference AAV (selection process).

Here, "a position corresponding to position 129 of the amino acid sequence of SEQ ID NO: 1 in the alignment with the amino acid sequence of SEQ ID NO: 1", means a position corresponding to position 129 of the amino acid sequence of SEQ ID NO: 1 in a case where the amino acid sequence of the capsid sequence is aligned with the amino acid sequence of SEQ ID NO: 1 in the "capsid sequence of reference AAV" into which a substitution of an amino acid residue is to be introduced. As described above, the "reference AAV" is not particularly limited, but may be a wild-type AAV or a wild-type AAV of serotype AAV1-10. Thus, the "capsid sequence of a reference AAV" may be, for example, the capsid sequence of a wild-type AAV and the capsid sequence of a wild-type AAV of serotype AAV1-10.

The alignment is performed by aligning the two amino acid sequences so that the amino acid residues of the two amino acid sequences to be compared match as much as possible. At the time of alignment, a gap is appropriately inserted into one or both of the two amino acid sequences to be compared, as necessary. Such alignment of amino acid sequences can be performed using a well-known program such as BLAST, FASTA, or CLUSTALW.

In addition, the amino acid residue at the position corresponding to the amino acid residue at the specific position may be an amino acid residue corresponding to the positions (1) to (8) of SEQ ID NO: 1 in AAV1 to AAV10, but is preferably an amino acid residue corresponding to the positions (1) to (8) of SEQ ID NO: 1 in AAV6, AAV7, or AAV8, and more preferably an amino acid residue corresponding to the positions (1) to (8) of SEQ ID NO: 1 in AAV6 or AAV8. The capsid sequence of AAV1 is shown in SEQ ID NO: 1. The capsid sequence of AAV2-10 is shown in SEQ IDs NO: 2 to 10. In addition, the alignment of the capsid sequences of AAV1-10 is also illustrated in Figs. 1-1 to 1-8.

As illustrated in Fig. 1, (1) leucine present at a position corresponding to position 129 of the amino acid sequence of SEQ ID NO: 1, (2) serine present at a position corresponding to position 268, (3) asparagine present at a position corresponding to position 447, (4) glycine present at a position corresponding to position 470, (5) serine present at a position corresponding to position 472, (6) valine present at a position corresponding to position 473, (7) threonine present at a position corresponding to position 502, and (8) glutamic acid present at a position corresponding to position 531 are (2-1) leucine at position 129, (2-2) asparagine present at position 268, (2-3) serine present at position 446, and (2-4) aspartic acid present at position 469, (2-5) arginine present at position 471, (2-6) aspartic acid present at position 472, (2-7) serine present at position 501, and (2-8) glutamic acid present at position 530 in the capsid sequence of AAV2 (SEQ ID NO: 2).

In addition, in the capsid sequence of AAV3 (SEQ ID NO: 3), there are (3-1) leucine at position 129, (3-2) asparagine at position 268, (3-3) asparagine at position 446, (3-4) serine at position 470, (3-5) serine at position 472, (3-6) leucine at position 473, (3-7) proline at position 502, and (3-8) glutamic acid present at position 531, respectively.

In the capsid sequence of AAV4 (SEQ ID NO: 4), there are (4-1) leucine at position 128, (4-2) serine at position 257, (4-3) glutamine at position 450, (4-4) asparagine at position 464, (4-5) serine at position 466, (4-6) asparagine at position 467, (4-7) lysine at position 503, and (4-8) aspartic acid at position 530, respectively.

In the capsid sequence of AAV5 (SEQ ID NO: 5), there are (5-1) phenylalanine at position 128, (5-2) serine at position 258, (5-3) valine at position 439, (5-4) no amino acid corresponding to position 470 of AAV1, (5-5) no amino acid corresponding to position 472 of AAV1, (5-6) no amino acid corresponding to position 473 of AAV1, (5-7) alanine at position 488, and (5-8) serine at position 518, respectively.

In the capsid sequence of AAV6 (SEQ ID NO: 6), there are (6-1) phenylalanine at position 129, (6-2) serine at position 268, (6-3) asparagine at position 447, (6-4) glycine at position 470, (6-5) serine at position 472, (6-6) valine at position 473, (6-7) threonine at position 502, and (6-8) lysine at position 531, respectively.

In the capsid sequence of AAV7 (SEQ ID NO: 7), there are (7-1) leucine at position 129, (7-2) threonine at position 269, alanine at position (7-3) 448, (7-4) threonine at position 472, (7-5) alanine at position 474, (7-6) glutamic acid at position 475, (7-7) alanine at position 504, and (7-8) glutamic acid at position 533, respectively.

In the capsid sequence of AAV8 (SEQ ID NO: 8), there are (8-1) leucine at position 129, (8-2) serine at position 268, (8-3) valine at position 448, (8-4) serine at position 470, (8-5) alanine at position 472, (8-6) asparagine at position 473, (8-7) alanine at position 502, and (8-8) glutamic acid at position 531, respectively.

In the capsid sequence of AAV9 (SEQ ID NO: 9), there are (9-1) leucine at position 129, (9-2) serine at position 268, (9-3) serine at position 448, (9-4) asparagine at position 470, (9-5) alanine at position 472, (9-6) valine at position 473, (9-7) alanine at position 502, and (9-8) glutamic acid at position 531, respectively.

In the capsid sequence of AAV10 (SEQ ID NO: 10), there are (10-1) leucine at position 129, (10-2) serine at position 269, (10-3) serine at position 449, (10-4) asparagine at position 472, (10-5) serine at position 474, (10-6) alanine at position 475, (10-7) alanine at position 504, and (10-8) glutamic acid at position 533, respectively.

In a case where an AAV other than AAV1-10 is used as the "reference AAV", similarly, by aligning the amino acid sequence of the capsid sequence of the AAV with the amino acid sequence of SEQ ID NO: 1, (1) leucine present at the position corresponding to position 129 of the amino acid sequence of SEQ ID NO: 1, (2) serine present at the position corresponding to position 268, (3) asparagine present at the position corresponding to position 447, (4) glycine present at the position corresponding to position 470, (5) serine present at the position corresponding to position 472, (6) valine present at the position corresponding to position 473, (7) threonine present at the position corresponding to position 502, and (8) glutamic acid present at the position corresponding to position 531 can be determined.

A further preferred modified AAV vector of the present invention is a vector in which the substitution of an amino acid residue at the positions of the above (1) to (8) in SEQ ID NO: 1 or a position corresponding thereto is one or more selected from Leu129Phe, Ser268Thr, Asn447Ser, Gly470Thr, Ser472Ala, Val473Asn, Thr502Ala, and Glu531Lys.

The modified AAV vector of the present invention has high infectivity to hair cells (inner hair cells, outer hair cells). In particular, modified AAV vectors having a combination of substitutions of Ser268Thr, Asn447Ser, Gly470Thr, Ser472Ala, Val473Asn, and Thr502Ala have high infectivity to hair cells. In addition, modified AAV vectors having a substitution of Leu129Phe or Glu531Lys in combination with these substitutions also have high infectivity to hair cells. Furthermore, modified AAV vectors having a combination of substitutions of Asn447Ser, Gly470Thr, Ser472Ala, Val473Asn, and Thr502Ala have higher infectivity to hair cells and significantly higher infectivity to outer spiral groove cells.

As the modification (recombination) means, normal gene targeting means can be used. Specifically, gene targeting means for adding a mutation to a PCR primer as described in Nat Methods 6, 343-345 (2009) can be employed.

The producing method of a modified adeno-associated virus vector having improved infection directivity to a target cell of the present invention is preferably a method including a process of substituting any one or more amino acid residues selected from the following (a) with other amino acid residues in the amino acid sequence of the capsid sequence.
(a) in alignment with the amino acid sequence of the capsid sequence of AAV1 of SEQ ID NO: 1, an amino acid residue at a position corresponding to
   (1) position 129 (Leu),
   (2) position 268 (Ser),
   (3) position 447 (Asn),
   (4) position 470 (Gly),
   (5) position 472 (Ser),
   (6) position 473 (Val),
   (7) position 502 (Thr), and
   (8) position 531 (Glu).

More specifically, the producing method preferably includes
a process of substituting any one or more amino acid residues selected from the following (a) with other amino acid residues in the amino acid sequence of the capsid sequence of a reference adeno-associated virus vector to prepare a candidate modified adeno-associated virus vector (candidate AAV preparation process),
   (a) in alignment with the amino acid sequence of the capsid sequence of AAV1 of SEQ ID NO: 1, an amino acid residue at a position corresponding to
      (1) position 129 (Leu),
      (2) position 268 (Ser),
      (3) position 447 (Asn),
      (4) position 470 (Gly),
      (5) position 472 (Ser),
      (6) position 473 (Val),
      (7) position 502 (Thr), and
      (8) position 531 (Glu);
a process of measuring an infectious dose of the candidate modified adeno-associated virus vector with the target cell (measurement process); and
a process of selecting a candidate modified adeno-associated virus vector in which the infectious dose with the target cell is increased as compared to the reference adeno-associated virus vector before introducing the amino acid substitution (selection process).

The reference adeno-associated virus vector is preferably AAV1, AAV6, AAV7, or AAV8, and more preferably AAV1, AAV6, or AAV8.

The amino acid substitution is more preferably one or more selected from Leu129Phe, Ser268Thr, Asn447Ser, Gly470Thr, Ser472Ala, Val473Asn, Thr502Ala, and Glu531Lys.

The measurement of the infectious dose to the target cell in the measurement process can be performed by measuring the number of cells expressing a gene (a gene encoding a protein such as a reporter protein) mounted on the candidate-modified AAV or the expression intensity of the protein in the cells.

The target cell may be an epithelial cell, a stromal cell, a lens cell, a corneal cell, a retinal cell, a skeletal muscle cell, a cardiomyocyte, a pancreatic cell, a hepatocyte, a neuronal cell, a neuroglia cell, a blood cell, a chondrocyte, an osteocyte, a fibroblast, an adipocyte, or a cancer cell.

The epithelial cells may be inner-ear epithelial cells, intestinal epithelial cells, retinal cells, skin cells, renal cells, airway epithelial cells, oral mucosal cells, nasal mucosal cells, glomerular cells, bladder cells, or uterine cells.

Furthermore, the target cell may be, in particular, a cell having a gap junction or expressing a connexin molecule to be a component of the gap junction.

The resulting modified AAV vectors have significantly improved infection directivity to target cells, for example, the epithelial cells.

For example, a modified AAV vector having one or two or more substitutions selected from Ser268Thr, Asn447Ser, Gly470Thr, Ser472Ala, Val473Asn, and Thr502Ala for the capsid sequence of AAV1 has high specificity for outer hair cells while wild-type AAV1 hardly infects hair cells (Inner hair cells, outer hair cells), and has the highest infectivity to outer hair cells among the inner-ear epithelial cell group, resulting in an infection efficiency of 80 to 100%.

Any combination of these amino acid substitutions has the potential to create modified AAV with infection directivity to a variety of target cells.

Specific examples of such modified AAV vectors include AAV vectors having a capsid sequence of the amino acid sequence of SEQ ID NO: 15 (referred to in the disclosure as "AAV-sia1.8").

In addition, the AAV vector having the capsid sequence of the amino acid sequence of SEQ ID NO: 16 (Glu531Lys variant of AAV-sia1.8) and the AAV vector having the capsid sequence of the amino acid sequence of SEQ ID NO: 17 (Leu129Phe variant of AAV-sia1.8), which are further variants of the same modified AAV vector, are more infectious to outer hair cells and support cells than AAV1, and form a large number of gap junction complexes having a large area in support cells. Some inner hair cells are also infected.

In addition, modified AAV vectors having one or two or more substitutions selected from Ser268Thr, Asn447Ser, Gly470Thr, Ser472Ala, Val473Asn, and Thr502Ala with respect to the capsid sequence of AAV6 have high infectivity to outer hair cells, similarly to modified AAV vectors having the same substitutions with respect to the capsid sequence of AAV1. Specificity to outer hair cells and infection directivity are weaker than the modified form of AAV1, but it has the ability to infect more extensively than the modified form of AAV1.

Any combination of these amino acid substitutions has the potential to create modified AAV with infection directivity to a variety of target cells.

Specific examples of such modified AAV vectors include AAV vectors having a capsid sequence of the amino acid sequence of SEQ ID NO: 14 (AAV-sia6.8).

In addition, an AAV vector that is a further variant of the modified AAV vector and has a capsid sequence of the amino acid sequence of SEQ ID NO: 18 (Thr268Ser variant of AAV-sia6.8) has higher infectivity to hair cells and significantly higher infectivity to outer spiral groove cells than AAV-sia6.8.

The modified AAV vectors of the present invention have improved infection directivity for a variety of epithelial cells, particularly epithelial cells that form gap junctions. Specific epithelial cells include epithelial cells selected from inner-ear epithelial cells, intestinal epithelial cells, retinal cells, skin cells, renal cells, airway epithelial cells, neuronal cells, cardiomyocytes, and cancer cells in which Connexin expression is involved.

Therefore, the modified AAV vectors of the present invention are useful for gene therapy and genome editing therapy for diseases in these epithelial cells.

Since the modified AAV vector of the present invention has improved infection directivity to the target cell, it is preferable to incorporate a gene that can be applied to gene therapy and genome editing therapy for genetic diseases, tissue damage, and cancer in the target cell into the modified AAV vector. Examples of such genes include causative genes of genetic diseases such as GJB2 gene, SLC26A4 gene, TMC1 gene, TMC2 gene, KCNQ4 gene, CHD23 gene, protocadherin 15 (PCDH15) gene, and otoferlin (OTOF) gene; and the transcription factor genes involved in cell differentiation and proliferation, such as Atoh1 gene, CDKN1B gene, and Hes1 gene.

Additional examples of genes particularly associated with genetic deafness include:
ACTG1, ADCY1, ADGRV1, AIFM1, BDP1, BSND, BTD, CABP2, CCDC50, CD164, CDC14A, CDH23, CEACAM16, CIB2, CLDN14, CLIC5, CLRN1, COCH, COL11A1, COL11A2, COL2A1, COL4A3, COL4A4, COL4A5, COL4A6, COL9A1, COL9A2, COL9A3, DCDC2, DIAPH1, DMXL2, DSPP, EDN3, EDNRB, ELM0D3, EPS8, EPS8L2, ESPN, ESRRB, EYA1, EYA4, FAM189A2, GIPC3, GJB3, GJB6, GPSM2, GRHL2, GRXCR1, GRXCR2, GSDME, HARS1, HGF, H0MER2, ILDR1, KARS1, KCNE1, KCNQ1, LHFPL5, LOXHD1, LRTOMT, MARVELD2, MCM2, MET, MIR96, MITF, MSRB3, MT-C01, MT-RNR1, MT-TS1, MYH14, MYH9, MYO15A, MYO1A, MYO3A, MYO6, MYO7A, MYO7A, NARS2, NF2, 0SBPL2, OTOA, OTOG, OTOGL, P2RX2, PAX3, PEX7, PHYH, PJVK, PNPT1, POU3F4, POU4F3, PRPS1, PTPRQ, RDX, RIPOR2, ROR1, S1PR2, SERPINB6, SIX1, SIX5, SLC17A8, SLC22A4, SLC26A5, SMPX, SOX10, STRC, SYNE4, TBC1D24, TECTA, TIMM8A, TJP2, TMEM132E, TMIE, TMPRSS3, TPRN, TRIO, TSPEAR, USH1C, USH1G, USH2A, WBP2, WFS1, and WHRN.

Modified AAV vectors of the present invention may incorporate promoters for expression of the aforementioned genes. As the promoter, a known promoter such as CMV or CAG can be used, but for a promoter for a gene in a GJB2 expressing cell such as an inner-ear supporting cell, in particular, a promoter containing any base sequence of SEQ ID NOs: 11 to 13 can be suitably used. One promoter may be continuously and repeatedly arranged a plurality of times (tandem of a single promoter), or any or all promoters may be continuously or discontinuously and repeatedly arranged a plurality of times by combining two or more promoters (tandem of a plurality of promoters).

In addition, in the modified AAV vector of the present invention, an enhancer for enhancing the activity of a promoter can be incorporated. As the enhancer, a known enhancer can be used, and examples of an enhancer suitably combined with a promoter containing the base sequence of SEQ ID NOs: 11 to 13 include an enhancer containing the base sequence of SEQ ID NO: 19 ("Characterization of GJB2 cis regulatory elements in the DFNB1 locus", Human Genetics (2019) 138:1275-1286). An enhancer may be arranged multiple times in succession (tandem of single enhancers), or two or more may be combined such that any or all enhancers are arranged multiple times in succession or discontinuity (tandem of multiple enhancers).

Preferred specific examples of gene therapy using the modified AAV vectors of the present invention include gene therapy to GJB2 mutant deafness, which is the most prevalent in genetic deafness. That is, the most common genetic deafness is GJB mutant deafness, accounting for about 45% of all cases of congenital deafness. A deafness gene therapeutic agent in which a normal GJB gene is incorporated into a modified AAV vector of the present invention can be produced. This gene therapeutic agent is administered by local administration to the inner-ear lymphatic fluid from the inner-ear footbone bottom plate, intravenous administration, or the like, and the abnormal gene function is normalized by efficiently expressing the target gene in the target cell.

Administration may be intravenous, intracerebroventricular, intra-cochlear, intrathecal, intramuscular, subcutaneous, or a combination thereof. In particular, the administration may be to the cochlea or vestibular system and the delivery thereof may be injection into the cochlea or vestibular system via the round window membrane (RWM), the oval window, or the semicircular canal.

The gene therapeutic agent and the genome editing therapeutic agent of the present invention can be formulated by mixing, dissolving, emulsifying, encapsulating, lyophilizing or the like the modified AAV vector together with a pharmaceutically acceptable carrier well known in the art.

Suitable formulations for oral administration are solutions obtained by dissolving an effective amount of the modified AAV vector of the present invention in a diluent such as water or physiological saline, capsules containing an effective amount as a solid or granule, granules, powders or tablets, suspensions obtained by suspending an effective amount in an appropriate dispersion medium, emulsions obtained by dispersing a solution obtained by dissolving an effective amount in an appropriate dispersion medium and emulsifying the dispersion medium, and the like.

For parenteral administration, the modified AAV vector of the present invention can be formulated into a dosage form such as an injection solution, a suspension, an emulsion, a cream, an ointment, an inhalant, or a suppository, together with a pharmaceutically acceptable solvent, an excipient, a binder, a stabilizer, a dispersant, or the like. In injectable formulations, the modified AAV vectors of the present invention can be dissolved in an aqueous solution, preferably a physiologically compatible buffer such as a Hanks solution, a Ringer's solution, or a physiological saline buffer. Furthermore, the medicament of the present invention can take the form of a suspension, solution, emulsion or the like in an oily or aqueous vehicle. Alternatively, the modified AAV vector of the present invention may be produced in the form of a powder, and an aqueous solution or a suspension may be prepared using sterile water or the like before use. For administration by inhalation, the modified AAV vectors of the present invention can be powdered into a powder mixture with a suitable base such as lactose or starch. The suppository formulation can be produced by mixing the inventive modified AAV vector with a conventional suppository base such as cocoa butter. Furthermore, the therapeutic agent of the present invention can be enclosed in a polymer matrix or the like and formulated as a sustained release formulation.

The dose of the modified AAV vector of the present invention varies depending on the patient's symptoms, administration route, body weight, age, and the like, but is preferably, for example, 0.5 µg to 100 mg per day for an adult.

### Examples

Next, the present invention will be described in more detail with reference to examples, but the present invention is not limited to these examples.

### Example 1: Evaluation of Infection Directivity of Reference AAV (Cultured Inner-ear Epithelial Tissue)

Using AAV vectors of various serotypes (GeneCapoeia, AAVPrime^{™} Adeno-associated virus (AAV) Serotype Testing Kit Plus, Cat. No. AA 321), the infection directivity to the inner-ear epithelial cells was examined by the method described in YODOSHA CO., LTD., Experimental Medicine Special Edition

Definitive edition, Guide to gene transfer experiments using viral vectors, Gene therapy for the inner ear using AAV vectors, Pages 159-166 (2020.11).

In the AAV vector, GJB2 gene encoding connexin 26 was incorporated under the control of a promoter (referred to as Promoter 1, Promoter 2, and Promoter 3, respectively) containing any base sequence of SEQ ID NOs: 11 to 13.

The inner ear was extracted from 4-day-old mice (C57BL/6 J system), and tissues containing sensory epithelium were cultured overnight in DMEM medium on 8-well chamber slides (Matsunami, Cat. No. SCS-N 38). Each serotype AAV(AAV1-10, 5 × 10⁸-5 × 10⁹ GC) was added the next day. The cells were cultured for 4 to 6 days, fixed with 4% paraformaldehyde, then immunofluorescence stained with an anti-connexin 26 antibody (Thermofisher Scientific. Cat. No. 51-2800), and observed with a fluorescence microscope together with green fluorescence of EGFP, which is a reporter gene of AAV, to measure cells forming a gap junction between EGFP positive cells and connexin 26.

As a result, as illustrated in Figs. 2 and 3, it was found that AAV1, AAV6, AAV7, and AAV8 have a higher infection directivity to inner-ear cells, particularly supporting cells forming gap junctions, than other serotypes.

In addition, Promoter 1 (a gene sequence of 1065bp upstream from the transcription start point of the human GJB2 gene), Promoter 2 (a gene sequence of 760bp upstream from the transcription start point of the human GJB2 gene), and Promoter 3 (a gene sequence of 260bp upstream from the transcription start point of the human GJB2 gene) were suitable for expression of various proteins in inner-ear supporting cells and inner-ear fiber cells. When the promoter activity was evaluated based on the expression intensity of connexin 26, Promoter 1, Promoter 2, and Promoter 3 were found in descending order of activity.

### Example 2: Preparation of Modified AAV by Introduction of Amino Acid Substitution into Reference AAV and Evaluation of Infection Directivity (Cultured Inner-ear Epithelial Tissue)

A vector in which various amino acid substitutions were introduced into the amino acid sequence of the capsid sequence was prepared using gene targeting means for adding a mutation to the PCR primer described in Nat Methods 6, 343-345 (2009). A GJB2 gene and a reporter gene (EGFP gene) were also introduced into the modified vector.

Changes in transduction efficiency and infection directivity of the resulting modified vectors are shown in Table 1.

As a result, it was found that the modified AAV vector having substitutions of Ser268Thr, Asn447Ser, Gly470Thr, Ser472Ala, Val473Asn, and Thr502Ala with respect to the capsid sequence of AAV1 (AAV-sia1.8: SEQ ID NO: 15) has high specificity for outer hair cells while AAV1 hardly infects hair cells (inner hair cells, outer hair cells), and has the highest infectivity to outer hair cells in the inner-ear epithelial cell group, resulting in an infection efficiency of 80% to 100%.

In addition, a modified AAV vector (AAV-sia6.8: SEQ ID NO: 14) having substitutions of Ser268Thr, Asn447Ser, Gly470Thr, Ser472Ala, Val473Asn, and Thr502Ala for the capsid sequence of AAV6 has high infectivity to outer hair cells as in the case of AAV-sia1.8. It was found that although the specificity to the outer hair cells and the infection directivity were weaker than those of AAV-sia1.8, the inner-ear epithelial cell group was more widespread than AAV-sia1.8.

**[Table 1]**

| Engineered AAV | Reference AAV | Substitution | Transduction efficiency | Change in infection orientation |
|---|---|---|---|---|
| AAV-Sia 1.8 | AAV1 | Ser268Thr, Asn447Ser, Gly470Thr, Ser472Ala, Val473Asn, Thr502Ala | - | OHC from OSC |
| AAV-Sia 6.8 | AAV6 | Ser268Thr, Asn447Ser, Gly470Thr, Ser472Ala, Val473Asn, Thr502Ala | ↑ ↑ | OHC/IHC/OSC from OSC |

| | | | | |
|---|---|---|---|---|
| "↑↑": Increased by 50% or more "↑": Increased by 1% to less than 50% "↓": Decreased by 1% to less than 50% "↓↓": Increased by 50% or more "OSC": Outer Sulcus Cell "OHC": Outer Hair Cell "IHC": Inner Hair Cell | | | | |

### Example 3: Introduction of Additional Amino Acid Substitutions into Modified AAV and Evaluation of Infection Directivity

A Glu531Lys variant of AAV-sia1.8 and a Leu129Phe variant of AAV-sia1.8 were prepared in the same manner as in Example 2.

A Glu531Lys variant of AAV-sia1.8 (SEQ ID NO: 16) is more infectious to outer hair cells and supporting cells than AAV1, and forms many gap junction complexes having a large area in the supporting cells. Some inner hair cells are also infected.

Similarly, a Leu129Phe variant of AAV-sia1.8 (SEQ ID NO: 17) is more infectious to outer hair cells and supporting cells than AAV1, and forms many gap junction complexes having a large area in the supporting cells. Some inner hair cells are also infected.

### Example 4: Gene Introduction into Cultured Inner-ear Epithelial Tissue derived from Deafness Model Animal

Fig. 4 illustrates a stained image in which GJB2 (GJB2 and EGFP introduced) was introduced into the inner-ear epithelium of a deafness model animal (GJB2 deficient mouse) by AAV sia1.8. Red indicates connexin 26 protein by introduced GJB2 gene. Green indicates EGFP reporter. EGFP expression indicating infection is specifically strong in the outer hair cells, and EGFP expression indicating infection to other inner hair cells, supporting cells, and the like and expression of connexin 26 are small.

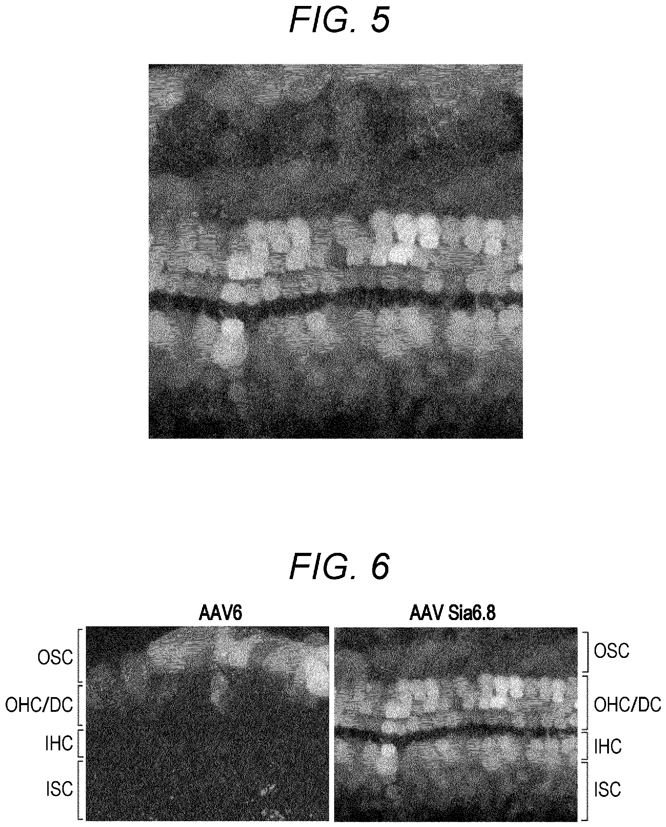

Fig. 5 illustrates a stained image in which GJB2 (GJB2 and EGFP introduced) was introduced into the inner ear epithelium of the GJB2-deficient mouse by AAV-sia6.8. Red indicates gap junction including connexin 26 by introduced GJB2 gene. Green indicates EGFP reporter. Portions with red lines between cells are gap junctions formed by gene therapy.

### Example 5: Gene Introduction into Cultured Cochlear Organ derived from Deafness Model Animal

Using cultured cochlear organs of GJB2 deficient mice, an infection experiment with wild-type AAV6 and AAV-sia6.8 carrying GJB2 gene and EGFP gene was performed.

Four days after birth, mice were experimentally killed under anesthesia, and cochlea was removed from temporal bone. The cochlear axis was removed from the cochlea, and the stria vascularis was removed from the organ of Corti. Corti organs were transferred to 8-well chamber slides and cultured (5% CO₂ atmosphere, 37°C) overnight in Dulbecco's Modified Eagle's Medium (Addition of high glucose, sodium pyruvate, GlutaMAXTM Supplement, fetal bovine serum, N2 Supplement, and penicillin G potassium). The media was then replaced with media containing 4 ul of AAV solution per 100 µl (2.05 × 10¹³ GC/mL). After a further 5 days of culture, the organ of Corti was treated with 4% paraformaldehyde and fixed.

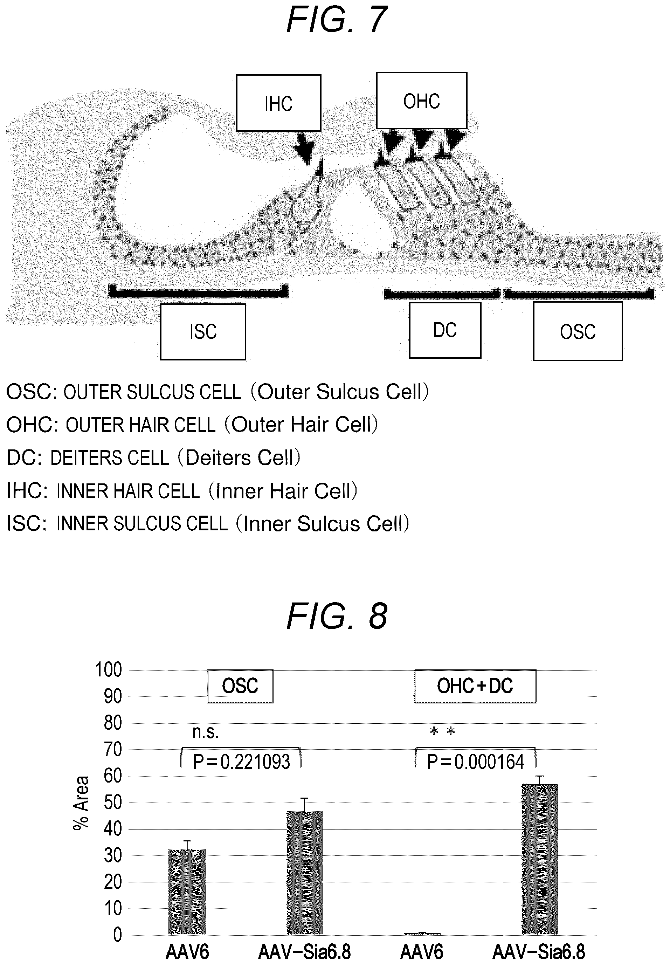

The results are illustrated in Fig. 6. In wild-type AAV6, EGFP signal was detected primarily in outer spiral groove cells (OSC). On the other hand, in AAV-sia6.8, the EGFP signal was detected widely in the entire inner-ear epithelium (OSC, OHC, IHC, DC, ISC) (see also Fig. 7).

Fig. 8 illustrates the results of image analysis of the EGFP positive area ratio in the region of the outer spiral groove cells (OSC) and the region containing the outer hair cells and the Diterus cells (OHC+DC). It was shown that AAV-sia6.8 has a higher positive area ratio in both the region of the outer spiral groove cells (OSC) and the region containing the outer hair cells and the vitreous cells (OHC+DC) than wild-type AAV6, and can perform gene introduction into a wide range of cells of the inner-ear epithelial cells with high efficiency.

### Example 6: Gene Therapy (Repair of Gap Junctions in Inner-ear Epithelial Cells derived from Deaf Patient-derived iPSC cells)

The differentiation of inner-ear epithelial cells was induced from induced pluripotent stem cells (iPSCs) established from patients with severe deafness due to abnormality of GJB2 gene and persons with normal hearing. AAV-sia6.8 carrying a wild-type GJB2 gene was infected with inner-ear epithelial cells, and gap junction repair by expression of connexin 26 (CX26) was performed.

An iPSC strain derived from a healthy subject (201B7) was obtained from RIKEN BioResource Center Cell Bank.

An iPSC line derived from a hearing-impaired patient (cell line name: JUFMDOi005-A) was created by the method described in an academic paper by the present inventors ("Generation of two induced pluripotent stem cell lines from PBMCs of siblings carrying c.235delC mutation in the GJB2 gene associated with sensorineural hearing loss", Stem Cell Research, Volume 47, August 2020, 101910).

iPSCs were suspended in Stem Fit medium supplemented with 20 um Y27632 and seeded into 96 well plates at 9000 cells/well. After 2 days of incubation (37°C, 5% CO₂), cell aggregates were transferred to a 96-well plate containing 100 ul of gfCDM medium (Ham's F12 GlutaMax/IMDM GlutaMax (1:1) (Gibco), 1% Chemically defined lipid concentrate (Gibco), 0.5% BSA (Sigma), 15 ug/ml Transferin (Sigma), 450 µM 1-Thioglycerol (Sigma)) supplemented with 2% Matrigel for further incubation. After 7 to 11 days, cell aggregates were half-split using forceps. The divided cell aggregates were transferred onto mitomycin C-treated inner ear-derived feeder cells (trypsin-resistant inner ear cells: TRIC) in DFNB medium (DMEM/F12 (1:1) (Gibco), 1% N2 supplement (Gibco), 1% B27 supplement (Gibco)). 20 to 25 days after transferring the cell aggregates onto TRIC, the inner-ear epithelial cells are induced to differentiate.

TRIC was prepared from cochlear tissue collected from 10 week old mice. Cochlear tissue is composed of the organ of Corti, basement membrane, and side wall, and is mainly composed of cells of supporting cells, hair cells, cochlear nerve fiber cells, and other basement membranes. Cochlear tissue was exposed to trypsin and TRIC was obtained by screening trypsin-resistant cells. These cells were maintained under growth medium (DMEM-GlutaMax (Gibco), 100 FBS (Gibco)).

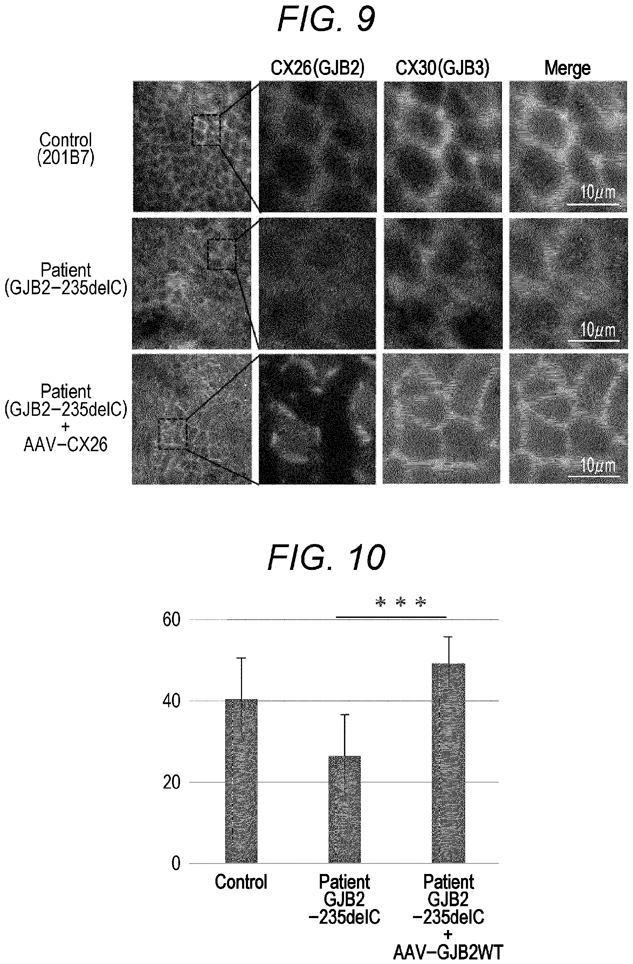

Expression of Connexin 26 (CX26) and Connexin 30 (CX30: GJB6 gene product) to be compared in inner-ear epithelial cells was detected by immunostaining. In addition, the expression intensity ratio of CX30 and CX26 was image-analyzed, and the expression intensity ratio of CX30 to CX26 was calculated. The results are illustrated in Figs. 9 and 10. In the inner-ear epithelial cells of healthy subjects (201B7), gap junctions positive for CX30 and CX26 were clearly observed, and CX26 was expressed with an intensity of about 40% relative to CX30. In the inner-ear epithelial cells of deafness patients (GJB2-235delC), the inner-ear gap junction was indistinct and insufficiently formed, and the expression intensity of CX26 to CX30 was also reduced to about 25%. In the inner-ear epithelial cells of the deafness patient into which the wild-type GJB2 gene was introduced by AAV-sia6.8, the expression intensity of CX26 to CX30 recovered to about 50% (GJB2-235delC+AAV-GJB2WT), and it was confirmed that the gap junction was repaired.

### Example 7: Gene Therapy (Hair Cell Regeneration)

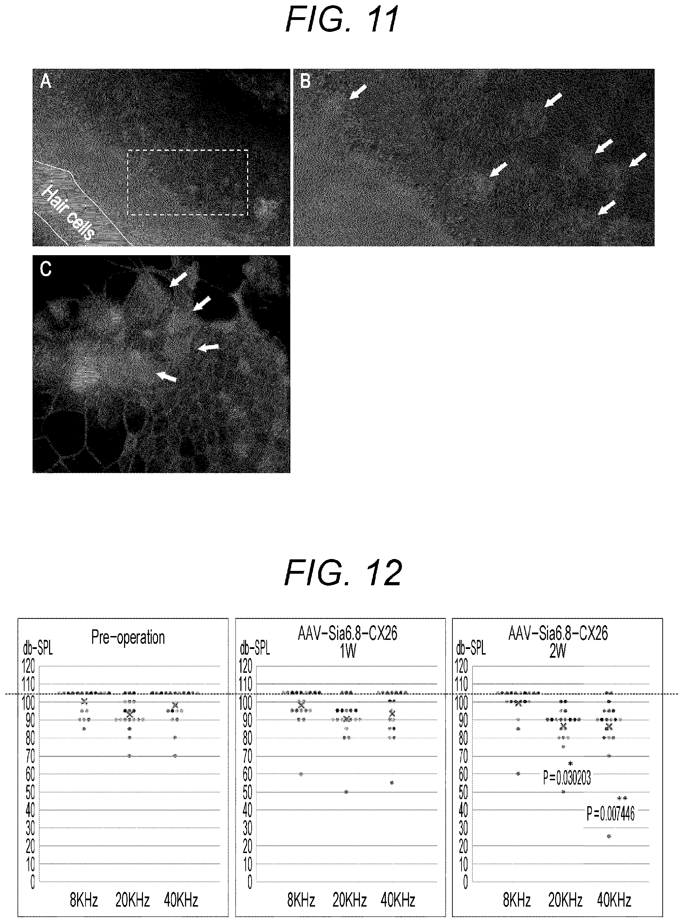

Using cultured cochlear organ of a mouse, an infection experiment of AAV-sia6.8 carrying protein atonal homologue 1 (Atoh1) gene was performed. The transcription factor Atoh1 is known to induce differentiation of a supporting cell into a hairy cell when being transfected into the supporting cell. As illustrated in Fig. 11, hair cell-like cells appeared in a region different from the hair cells region (a region surrounded by a dotted line in Fig. 11(A)) (see arrows in Figs. 11(B) and 11(C)). Fig. 11(B) is an enlarged view of a region surrounded by a dotted line in Fig. 11(A). The possibility that AAV-sia6.8 can be effectively utilized for regenerating hair cells when hair cells are deficient due to noise-induced deafness or the like has been shown.

### Example 8: Gene Therapy (Hearing Recovery of Deafness Model Animal)

The hearing of GJB2 deficient mice (3 to 4 weeks old) was measured based on auditory brainstem response (ABR) (pre-operative hearing). Under anesthesia, small holes were provided at two locations of the lateral semicircular canal of the mouse using a dental drill. A capillary tube was inserted into one hole, and an AAV-sia6.8 solution (about 1×10¹³ GC/ml) carrying 10 to 12 ul of wild-type GJB2 gene was injected into the endolymph fluid (endolymph) at a rate of 3 µl/min. One week and two weeks after surgery, hearing was measured based on auditory brainstem response (ABR) (postoperative hearing).

ABR measurements were performed by placing stainless steel needle electrodes at the top of the left and right ears and on the ventrolateral side using a Power Laboratory system (PowerLab 4/25; AD Instruments) Scope software waveform preservation and stimulation control. The electroencephalographic recording was performed using an extracellular amplifier AC PreAmplifier (Model P-55; Astro-Med). The acoustic stimulation was performed by using a coupler-type speaker (model ES1spc; Bio Research Center). The threshold value was determined based on a series of responses with varying intensity at 5dB intervals at frequencies of 8, 20, and 40 kHz.

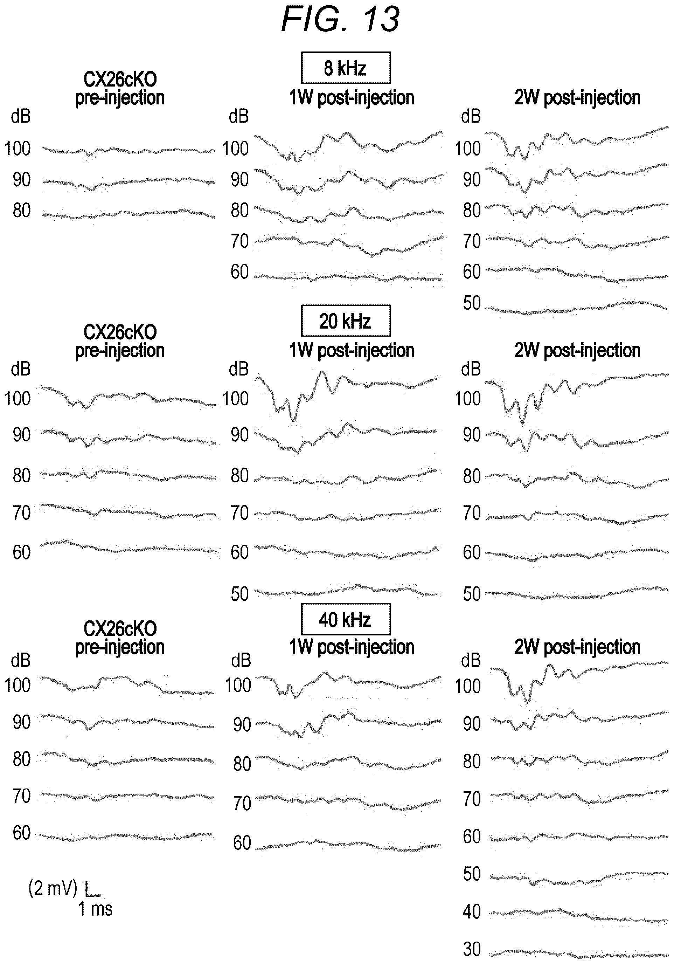

The threshold values and waveforms of the ABR before and after surgery are illustrated in Figs. 12 and 13, respectively. In 2 weeks after surgery (AAV-Sia6.8 CX26 2W), the threshold value of ABR at a high wavelength (20 kHz, 40 kHz) decreased as compared with pre-operation, and significant improvement in hearing was observed (see Fig. 12). In addition, in 2 weeks after surgery (2W post-infection), a reaction was confirmed even to a weak sound pressure that was not reacted before surgery (CX26cKO pre-injection) (see Fig. 13).

### Example 9: Cell Targeting Gene Transfer Using Promoter

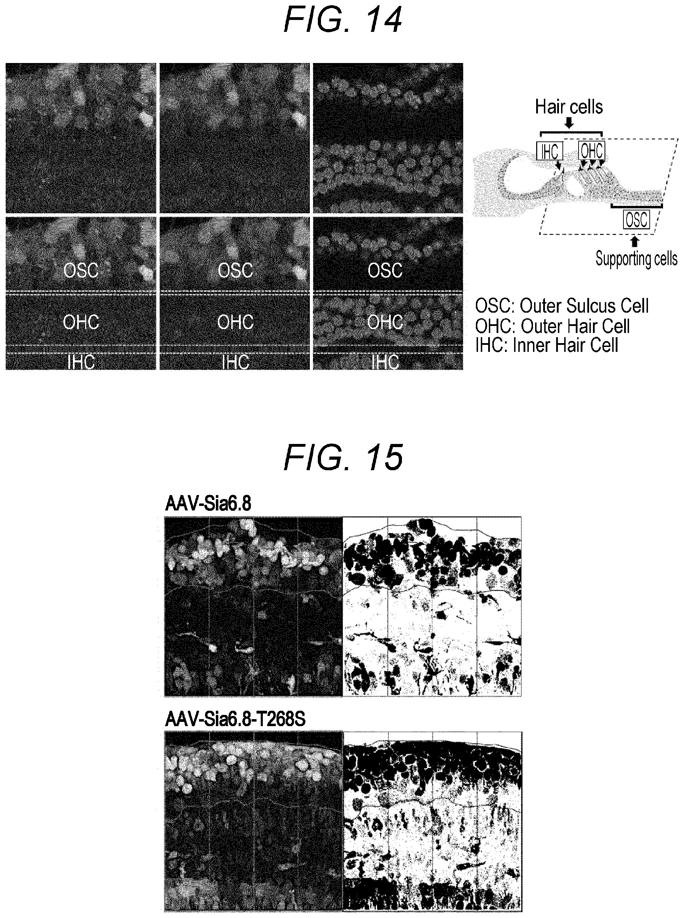

Promoter 2 (760bp gene sequence upstream from the transcription start point of the human GJB2 gene, SEQ ID NO: 12) was introduced into AAV-sia6.8, and GJB2 gene and EGFP gene were mounted thereon, and cultured cochlear organs of GJB2 deficient mice were infected. As illustrated in Fig. 14, formation of gap junctions with expression of EGPF (green) and expression of connexin 26 (red) was confirmed in the outer spiral groove cells (OSC) as supporting cells. No infection of outer and inner hair cells (OHC and IHC) was observed. This result showed that AAV-sia6.8 can be effectively used for cell targeting gene transfer (in this example, gene transfer targeting supporting cells) by introducing an appropriate promoter.

### Example 10: Introduction of Additional Amino Acid Substitutions into Modified AAV and Evaluation 2 of Infection Directivity

A Thr268Ser variant of AAV-sia6.8 was prepared in the same manner as in Example 2.

In the amino acid at position 268 of AAV-sia6.8, Ser in wild-type AAV6 is substituted with Thr. Thus, the Thr268Ser variant of AAV-sia6.8 is a modification that returns the amino acid at position 268 to the wild-type Ser.

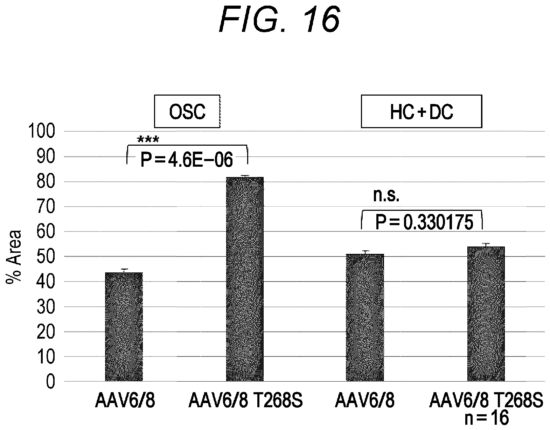

A mouse cultured cochlear organ was used to perform an infection experiment with a Thr268Ser variant of AAV-sia6.8 carrying the EGFP gene. The fluorescence image of EGFP and the results of calculating the EGFP positive area ratio by image analysis are illustrated in Figs. 15 and 16, respectively. In the Thr268Ser variant of AAV-sia6.8 (AAV-Sia-T268S), the infection directivity to inner-ear cells was improved as compared with AAV-sia6.8, and the infection efficiency was remarkably improved particularly in outer spiral groove cells (OSC).

### [Sequence Listing Free Text]

SEQ ID NO: 1: Amino acid sequence of capsid sequence of AAV1
SEQ ID NO: 2: Amino acid sequence of capsid sequence of AAV2
SEQ ID NO: 3: Amino acid sequence of capsid sequence of AAV3
SEQ ID NO: 4: Amino acid sequence of capsid sequence of AAV4
SEQ ID NO: 5: Amino acid sequence of capsid sequence of AAV5
SEQ ID NO: 6: Amino acid sequence of capsid sequence of AAV6
SEQ ID NO: 7: Amino acid sequence of capsid sequence of AAV7
SEQ ID NO: 8: Amino acid sequence of capsid sequence of AAV8
SEQ ID NO: 9: Amino acid sequence of capsid sequence of AAV9
SEQ ID NO: 10: Amino acid sequence of capsid sequence of AAV10
SEQ ID NO: 11: Nucleic acid sequence of Promoter 1
SEQ ID NO: 12: Nucleic acid sequence of Promoter 2
SEQ ID NO: 13: Nucleic acid sequence of Promoter 3
SEQ ID NO: 14: Amino acid sequence of capsid sequence of AAV-sia6.8
SEQ ID NO: 15: Amino acid sequence of capsid sequence of AAV-sia1.8
SEQ ID NO: 16: Amino acid sequence of capsid sequence of Glu531Lys variant of AAV-sia1.8
SEQ ID NO: 17: Amino acid sequence of capsid sequence of Leu129Phe variant of AAV-sia1.8
SEQ ID NO: 18: Amino acid sequence of capsid sequence of Thr268Ser variant of AAV-sia6.8
SEQ ID NO: 19: Nucleic acid sequence of Enhancer 1

## Claims

1. A modified adeno-associated virus vector exhibiting improved infectivity tropism for a target cell, wherein
a substitution of one or two or more amino acid residues selected from the following (1) to (8) is introduced into an amino acid sequence of a capsid sequence:
(1) a substitution of leucine present at a position corresponding to position 129 of the amino acid sequence of SEQ ID NO: 1 with another amino acid residue in alignment with the amino acid sequence of SEQ ID NO: 1,
(2) a substitution of serine present at a position corresponding to position 268 of the amino acid sequence of SEQ ID NO: 1 with another amino acid residue in alignment with the amino acid sequence of SEQ ID NO: 1,
(3) a substitution of an asparagine present at a position corresponding to position 447 of the amino acid sequence of SEQ ID NO: 1 with another amino acid residue in alignment with the amino acid sequence of SEQ ID NO: 1,
(4) a substitution of glycine present at a position corresponding to position 470 of the amino acid sequence of SEQ ID NO: 1 with another amino acid residue in alignment with the amino acid sequence of SEQ ID NO: 1,
(5) a substitution of serine present at the position corresponding to position 472 of the amino acid sequence of SEQ ID NO: 1 with another amino acid residue in alignment with the amino acid sequence of SEQ ID NO: 1,
(6) a substitution of valine present at the position corresponding to position 473 of the amino acid sequence of SEQ ID NO: 1 with another amino acid residue in alignment with the amino acid sequence of SEQ ID NO: 1,
(7) a substitution of a threonine present at the position corresponding to position 502 of the amino acid sequence of SEQ ID NO: 1 with another amino acid residue in alignment with the amino acid sequence of SEQ ID NO: 1, and
(8) a substitution of a glutamic acid present at the position corresponding to position 531 of the amino acid sequence of SEQ ID NO: 1 with another amino acid residue in alignment with the amino acid sequence of SEQ ID NO: 1.

2. The modified adeno-associated virus vector according to claim 1, wherein the substitution of the amino acid residues is
(1) a substitution of leucine present at a position corresponding to position 129 of the amino acid sequence of SEQ ID NO: 1 with phenylalanine in alignment with the amino acid sequence of SEQ ID NO: 1,
(2) a substitution of serine present at a position corresponding to position 268 of the amino acid sequence of SEQ ID NO: 1 with threonine in alignment with the amino acid sequence of SEQ ID NO: 1,
(3) a substitution of asparagine present at a position corresponding to position 447 of the amino acid sequence of SEQ ID NO: 1 with serine in alignment with the amino acid sequence of SEQ ID NO: 1,
(4) a substitution of glycine present at a position corresponding to position 470 of the amino acid sequence of SEQ ID NO: 1 with threonine in alignment with the amino acid sequence of SEQ ID NO: 1,
(5) a substitution of serine present at the position corresponding to position 472 of the amino acid sequence of SEQ ID NO: 1 with alanine in alignment with the amino acid sequence of SEQ ID NO: 1,
(6) a substitution of valine present at the position corresponding to position 473 of the amino acid sequence of SEQ ID NO: 1 with asparagine in alignment with the amino acid sequence of SEQ ID NO: 1,
(7) a substitution of threonine present at the position corresponding to position 502 of the amino acid sequence of SEQ ID NO: 1 with alanine in alignment with the amino acid sequence of SEQ ID NO: 1, or
(8) a substitution of glutamic acid present at the position corresponding to position 531 of the amino acid sequence of SEQ ID NO: 1 with leucine in alignment with the amino acid sequence of SEQ ID NO: 1.

3. The modified adeno-associated virus vector according to claim 1 or 2, comprising: substitutions of the amino acid residues of (2) to (7) above in combination.

4. The modified adeno-associated virus vector according to claim 3, comprising: a capsid sequence consisting of the amino acid sequence of SEQ ID NO: 14 or SEQ ID NO: 15.

5. The modified adeno-associated virus vector according to claim 3, further comprising: a substitution of the amino acid residue in (1) or (8) above.

6. The modified adeno-associated virus vector according to claim 5, comprising: a capsid sequence consisting of the amino acid sequence of SEQ ID NO: 16 or SEQ ID NO: 17.

7. The modified adeno-associated virus vector according to claim 1 or 2, comprising: substitutions of the amino acid residues of (3) to (7) above in combination.

8. The modified adeno-associated virus vector according to claim 7, comprising: a capsid sequence consisting of the amino acid sequence of SEQ ID NO: 18.

9. The modified adeno-associated virus vector according to any one of claims 1 to 6, wherein the target cell is an epithelial cell, a stromal cell, a lens cell, or a cancer cell.

10. The modified adeno-associated virus vector according to claim 9, wherein the epithelial cell is an inner-ear epithelial cell, an intestinal epithelial cell, a retinal cell, a skin cell, a renal cell, an airway epithelial cell, a neuronal cell, or a uterine cell.

11. The modified adeno-associated virus vector according to claim 9 or 10, wherein the target cell has a gap junction or expresses a connexin molecule constituting a gap junction.

12. The modified adeno-associated virus vector according to claim 11, wherein a gene causing a genetic disease, a gene involved in differentiation and/or proliferation of cells, or a gene for genome editing these genes is mounted to be expressible.

13. The modified adeno-associated virus vector according to claim 12, wherein the gene causing the genetic disease is selected from the group consisting of a connexin 26 (GJB2) gene, a pendrin (SLC26A4) gene, a TMC1 gene, a TMC2 gene, a voltage-dependent potassium channel subfamily Q member 4 (KCNQ4) gene, a cadherin 23 (CHD23) gene, a protocadherin 15 (PCDH15) gene, and an otoferlin (OTOF) gene.

14. The modified adeno-associated virus vector according to claim 12, wherein the gene involved in differentiation and/or proliferation of the cell is selected from the group consisting of a protein atonal homolog 1 (Atoh1) gene, a cyclin dependent kinase inhibitor 1B (CDKN1B) gene, and a basic helix-loop-helix type transcription factor (Hes1) gene.

15. The modified adeno-associated virus vector according to claim 13, wherein a promoter including the base sequence of any of SEQ ID NOs: 11 to 13 is incorporated upstream of the GJB2 gene, optionally together with an enhancer of SEQ ID NO: 19, to controllably express the GJB2 gene.

16. A medicament comprising: the modified adeno-associated virus vector according to any one of claims 1 to 15.

17. The medicament according to claim 16, for use in a treatment of a genetic disease, tissue damage and/or cancer, or in genome editing.

18. Use of the modified adeno-associated virus vector according to any one of claims 1 to 15 for a manufacture of a medicament for the treatment of a genetic disease, tissue damage and/or cancer, or genome editing.

19. A method of treating a genetic disease, tissue damage, and/or cancer, the method comprising: a step of administering the modified adeno-associated virus vector according to any one of claims 1 to 15 to a subject having a genetic disease, tissue damage, and/or cancer in a therapeutically effective amount.

20. A genome editing method comprising: a step of administering the modified adeno-associated virus vector according to any one of claims 1 to 15 to a subject in an effective amount.

21. A producing method of a modified adeno-associated virus vector exhibiting improved infectivity tropism for a target cell, the method comprising:
a process of introducing a substitution of one or two or more amino acid residues selected from the following (1) to (8) into an amino acid sequence of a capsid sequence:
(1) a substitution of leucine present at a position corresponding to position 129 of the amino acid sequence of SEQ ID NO: 1 with another amino acid residue in alignment with the amino acid sequence of SEQ ID NO: 1,
(2) a substitution of serine present at a position corresponding to position 268 of the amino acid sequence of SEQ ID NO: 1 with another amino acid residue in alignment with the amino acid sequence of SEQ ID NO: 1,
(3) a substitution of an asparagine present at a position corresponding to position 447 of the amino acid sequence of SEQ ID NO: 1 with another amino acid residue in alignment with the amino acid sequence of SEQ ID NO: 1,
(4) a substitution of glycine present at a position corresponding to position 470 of the amino acid sequence of SEQ ID NO: 1 with another amino acid residue in alignment with the amino acid sequence of SEQ ID NO: 1,
(5) a substitution of serine present at the position corresponding to position 472 of the amino acid sequence of SEQ ID NO: 1 with another amino acid residue in alignment with the amino acid sequence of SEQ ID NO: 1,
(6) a substitution of valine present at the position corresponding to position 473 of the amino acid sequence of SEQ ID NO: 1 with another amino acid residue in alignment with the amino acid sequence of SEQ ID NO: 1,
(7) a substitution of a threonine present at the position corresponding to position 502 of the amino acid sequence of SEQ ID NO: 1 with another amino acid residue in alignment with the amino acid sequence of SEQ ID NO: 1, and
(8) a substitution of a glutamic acid present at the position corresponding to position 531 of the amino acid sequence of SEQ ID NO: 1 with another amino acid residue in alignment with the amino acid sequence of SEQ ID NO: 1.

22. The producing method according to claim 21, the method comprising:
a process of introducing substitutions of one or two or more amino acid residues selected from the above (1) to (8) into the amino acid sequence of the capsid sequence of a reference adeno-associated virus vector to prepare a candidate adeno-associated virus vector;
a process of measuring an infectious dose of the target cell of the candidate adeno-associated virus vector; and
a process of selecting a candidate adeno-associated virus vector in which the infectious dose of the target cell is increased as compared to the reference adeno-associated virus vector.

23. The producing method according to claim 21, wherein the reference adeno-associated virus vector is AAV1 to AAV10, preferably AAV1, AAV6, AAV7, AAV8, more preferably AAV1 or AAV6, particularly preferably AAV1.
